# EUROPEAN PATENT SPECIFICATION

(11) **EP 3 754 664 B1**
(45) Date of publication and mention of the grant of the patent: **26.08.2026**
(21) Application number: 19210546.8
(22) Date of filing: 21.11.2019
(51) Int. Cl.: G16H 10/60, G16H 50/20

(54) **DISEASE SUFFERING PROBABILITY PREDICTION METHOD AND ELECTRONIC APPARATUS**
VERFAHREN ZUR VORHERSAGE DER ERKRANKUNGSWAHRSCHEINLICHKEIT UND ELEKTRONISCHE VORRICHTUNG
PROCÉDÉ DE PRÉDICTION DE LA PROBABILITÉ DE SOUFFRIR D'UNE MALADIE ET APPAREIL ÉLECTRONIQUE

(30) Priority: 19.06.2019 TW 108121317
(43) Date of publication of application: 23.12.2020
(73) Proprietor: Acer Incorporated, New Taipei City 221 (TW); National Yang-Ming University, Taipei City 112 (TW)
(72) Inventor: Chen, Pei-Jung, 221 New Taipei City (TW); Tsai, Tsung-Hsien, 221 New Taipei City (TW); Chen, Liang-Kung, 112 Taipei (TW); Peng, Li-Ning, 112 Taipei (TW)
(74) Representative: 2K Patent Partnerschaft mbB

(56) References cited:
- CN-A- 106 778 014
- CN-A- 108 206 058
- CN-A- 109 460 473
- CN-A- 109 859 854
- US-A1- 2019 172 587

## Description

### BACKGROUND OF THE INVENTION

### 1. Field of the Invention

The present invention relates to a disease suffering probability prediction method and an electronic apparatus.

### 2. Description of Related Art

In general, based on the experience of diagnosis, doctors can determine what disease is likely to cause a specific disease. For example, dementia more likely occurs after diabetes. However, at present, it is mostly a single disease risk study, and there is no effective way to know which diseases are more likely to cause a specific disease sequentially.

CN 106 778 014 A discloses a circulating neural network-based disease risk prediction method, which comprises the following steps: (1) using the diagnosed disease as a training sample, performing disease name distributed word vector training to obtain a word vector mapping matrix, and storing the word vector mapping matrix; (2) secondly, taking the diagnosed disease as a training sample, and carrying out cyclic neural network training to obtain a disease risk prediction model; (3) and taking each diagnosis disease in the patient history record as a test sample to be input into the disease risk prediction model to obtain a disease risk prediction result. The method utilizes the cyclic neural network and the distributed word vector expression embedding technology, solves the problems of too complex training model, high training cost, low training accuracy and the like caused by the characteristics of high dimensionality, sparse data, strong time sequence and the like of medical diagnosis data, and realizes the modeling process with time sequence for historical diseased information.

US 2019/172587 A1 discloses an apparatus for predicting a disease risk of a metabolic disorder. The apparatus includes: a machine learning model generating unit which generates a machine learning model which learns a degree of a relationship between at least one of a plurality of state variables and genetic information and a disease risk of metabolic disorders with the plurality of state variables including a living condition variable and a health condition variable of a patient with a metabolic disorder, generic information, and the disease risk of the metabolic disorders as inputs; an information input unit which receives a subject state variable and subject genetic information of the subject; and a disease risk predicting unit which predicts a subject disease risk of the subject by applying the subject state variable and the subject genetic information of the subject to the machine learning model.

### SUMMARY OF THE INVENTION

The present invention provides a disease suffering probability prediction method and an electronic apparatus, which can calculate information such as a proportion or probability of a specific disease suffered by a patient according to an order of diseases suffered by the patient.

The present invention provides a disease suffering probability prediction method according to appended claim 1, applied to an electronic apparatus according to appended claim 9. Advantageous embodiments are the subject of the dependent claims.

Based on the above, the disease suffering probability prediction method and the electronic apparatus of the present invention can find an order (also referred to as a path) of diseases suffered before suffering a specific disease (e.g., dementia) from history data. People with these paths will have a higher probability of suffering the foregoing specific disease in the future than those without them. In addition, the disease suffering probability prediction method of the present invention can further use the foregoing path to calculate information such as a proportion or probability of suffering a specific disease.

In order to make the aforementioned and other objectives and advantages of the present invention comprehensible, embodiments accompanied with figures are described in detail below.

### BRIEF DESCRIPTION OF THE DRAWINGS

Fig. 1 is a block diagram of an electronic apparatus in accordance with an embodiment of the present invention.
Fig. 2 is a flowchart of a method for filtering a path for predicting dementia using history data in accordance with an embodiment of the present invention.
Fig. 3 is a flowchart of a path filtering method in accordance with an embodiment of the present invention.
Fig. 4 is a flowchart of a multi-model variable filtering method in accordance with an embodiment of the present invention.
Fig. 5 is a flowchart of a disease suffering probability prediction method in accordance with an embodiment of the present invention.

### DESCRIPTION OF THE EMBODIMENTS

Reference will now be made in detail to the present preferred embodiments of the invention, examples of which are illustrated in the accompanying drawings. Wherever possible, the same reference numbers are used in the drawings and the description to refer to the same or like parts, components or steps.

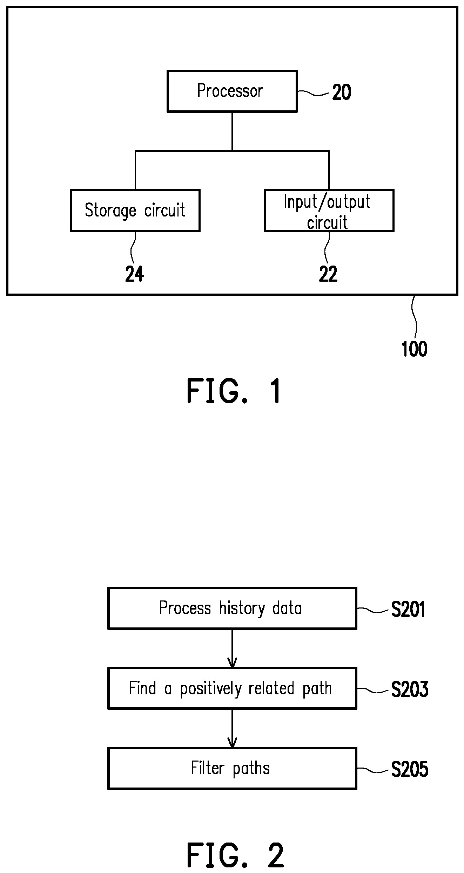

Fig. 1 is a block diagram of an electronic apparatus in accordance with an embodiment of the present invention.

Referring to Fig. 1, an electronic apparatus 100 includes a processor 20, an input/output circuit 22 and a storage circuit 24. The input/output circuit 22 and the storage circuit 24 are coupled to the processor 20 respectively. The electronic apparatus 100 is, for example, an electronic mobile apparatus such as a desktop computer, a server, a mobile phone, a tablet computer, or a notebook computer, which is not limited herein.

The processor 20 may be a central processing unit (CPU), or other programmable general-purpose or special-purpose microprocessors, digital signal processors (DSP), programmable controllers, application specific integrated circuits (ASIC), other similar components, or a combination of the aforementioned components.

The input/output circuit 22 is, for example, an input interface or circuit for obtaining related data from the outside of the electronic apparatus 100 or from other sources. In addition, the input/output circuit 22 may also transmit data generated by the electronic apparatus 100 to an output interface or circuit of another electronic apparatus, which is not limited herein.

The storage circuit 24 may be any type of fixed or mobile random access memory (RAM), read-only memory (ROM), flash memory or similar components, or a combination of the aforementioned components.

In the present exemplary embodiment, the storage circuit 24 of the electronic apparatus 100 stores a plurality of code segments, which are executed by the processor 20 after the code segments are installed. For example, the storage circuit 24 includes a plurality of modules by which respective operations of a disease suffering probability prediction method applied to the electronic apparatus 100 are respectively performed, where each module is composed of one or more code segments. However, the present invention is not limited thereto, and respective operations of the electronic apparatus 100 may be implemented by using other hardware forms.

It is to be noted that an order (also referred to as a path) of other diseases previously suffered by dementia patients may be related to future dementia before the diagnosis of dementia. The disease suffering probability prediction method of the present invention can find out the order of these diseases and provide information for doctors to assist in the prevention and treatment of dementia as a tool for assessing the risk of dementia. For example, a doctor can know which diseases are more susceptible to dementia sequentially, as well as the magnitude of a proportion or probability of dementia, etc.

In particular, the present invention is exemplified by dementia, but the present invention is not limited thereto. In other embodiments, the disease suffering probability prediction method of the present invention may also be applied to predict other diseases other than dementia, such as Parkinson's disease or other diseases, which are not limited herein. The following description is exemplified by dementia.

Fig. 2 is a flowchart of a method for filtering a path for predicting dementia using history data in accordance with an embodiment of the present invention. In the present exemplary embodiment, the definition of "path" is as follows: a combination of arranging several different diseases in an order, the order being an order conforming to time of the earliest occurrence of the diseases (or the earliest diagnosis). The definition of "path length" is as follows: a count of diseases in the foregoing path. In simple terms, the path may be diseases suffered (or diagnosed) by a patient sequentially, and the path length is a count of the diseases suffered by the patient.

Referring to Fig. 2, first, the processor 20 needs to perform a step of processing history data (step S201) to convert history data into data of the foregoing path.

In detail, the processor 20 needs to determine a path length according to requirements. Thereafter, the processor 20 may obtain a plurality of paths (hereinafter referred to as first paths) conforming to the path length from a plurality of history data of dementia according to the path length. In more detail, for each person in data, history data before suffering the dementia is taken, and then various diseases in the history are ranked at the time of the earliest occurrence (or time of the earliest diagnosis). From a disease ranking order of each person, all disease orders conforming to the foregoing path length are taken, and a relative disease order is maintained. All the paths that have appeared in the data are taken out.

The foregoing step of processing history data is exemplified below.

### [Example of Step S201]

It is assumed that the diagnosis history of patient A before dementia is: A->B->C->A->D->C. The foregoing A, B, C and D are diseases different from one other, and the diagnosis history may be a medical record of patient A. After obtaining the diagnosis history by the input/output circuit 22, the processor 20 may first rank the diseases according to time of the earliest diagnosis for each disease to obtain a disease ranking order: A->B->C->D. It is assumed that the previously determined path length is 3. The processor 20 may take out a combination of paths having a path length of 3 (i.e., composed of 3 diseases) from the foregoing disease ranking order and maintain a relative ranking between diseases, thereby obtaining the following 4 paths: A->B->C, A->B->D, A->C->D, and B->C->D.

It is assumed that all paths taken from the data of all patients (i.e., the foregoing first paths) are as follows: A->B->C, A->B->D, A->C->D, B->C->D, and B->A->C. These paths are taken as a feature, and the disease ranking order of patient A is converted into path data as shown in Table 1:

**Table 1**

| | A->B->C | A->B->D | A->C->D | B->C->D | B->A->C |
|---|---|---|---|---|---|
| Patient A | 1 | 1 | 1 | 1 | 0 |

Similarly, a path having a length of 3 may be obtained from data of each patient in the foregoing manner, and path data of each patient is recorded in Table 1 respectively.

Referring again to Fig. 2, next, the processor 20 performs a step of finding a positively related path (step S203). For example, the processor 20 may obtain a plurality of paths (also referred to as second paths) positively related to dementia from the first paths according to the foregoing plurality of first paths. In more detail, the processor 20 may calculate each path in the foregoing Table 1 separately using a machine learning method or a statistical method to determine whether a path is positively or negatively related to dementia. For example, the values of each column in the foregoing Table 1 are summed, paths summed to be greater than a threshold value are identified as positively related paths, and paths summed to be not greater than the threshold value are identified as negatively related paths. The processor 20 may preserve the positively related paths and delete the negatively related paths. In particular, a patient with a positively related path will have a higher chance of suffering dementia than a patient without a positively related path.

After performing step S203, the processor 20 performs a step of path filtering (step S205), thereby filtering the foregoing positively related paths (i.e., the second paths) to obtain a plurality of paths therefrom (also referred to as third paths). The processor 20 may establish a prediction model according to the third paths. In particular, this step mainly filters the foregoing second paths according to the prediction performance of dementia to find a third path with a better prediction ability from the second paths.

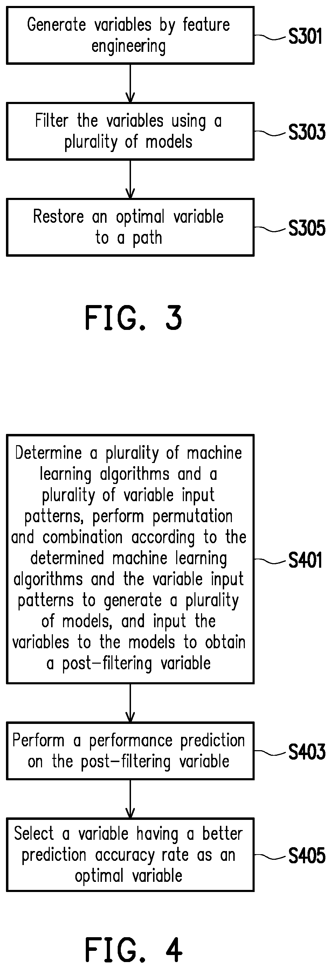

Fig. 3 is a flowchart of a path filtering method in accordance with an embodiment of the present invention.

Referring to Fig. 3, the foregoing step S205 may be further subdivided into steps S301 to S305. In more detail, in the process of performing step S205, the processor 20 generates a variable by feature engineering (step S301). For example, the processor 20 may generate a plurality of variables corresponding to a plurality of patterns according to the foregoing plurality of second paths.

In more detail, step S301 is to take out part or all of the diseases from each of the second paths and to generate a new variable accordingly. In particular, the generated new variable may have various different patterns by permutation and combination of a position of a disease, an order of diseases and a count of diseases.

Here, V(Count, Position, Order) is defined as the patterns of the new variable. "Count" represents a count of diseases taken from a path, which is equal to 1 minimally and equal to the length of the path maximally. "Position" represents a feature indicating whether a mode of taking a disease preserves a position of the disease in an original path, where the value thereof may be "Preserve Position (hereinafter referred to as PP)" or "Ignore Position (hereinafter referred to as IP)". "Order" represents whether a mode of taking diseases preserves an order of diseases, where the value thereof may be is "Preserve Order (hereinafter referred to as PO)" or "Ignore Order (hereinafter referred to as IO)". In particular, when the count of diseases is "1", the order is meaningless, and a threshold of "Order" may be set to "X".

A path length of 3 is taken as an example, where a new variable may have the following patterns (1) to (7).

| | |
|---|---|
| V(1, PP, X) | pattern (1) |
| V(1, IP, X) | pattern (2) |
| V(2, PP, PO) | pattern (3) |
| V(2, PP, IO) | pattern (4) |
| V(2, IP, PO) | pattern (5) |
| V(2, IP, IO) | pattern (6) |
| V(3, X, IO) | pattern (7) |

It is to be noted that in the case of a path, since the position is meaningless when the count is "3", the value of the "Position" field is X, and when the "Order" field is "PO", the original path is represented. Therefore, the pattern "V(3, X, PO)" is not recorded as a new variable.

In the present embodiment, the processor 20 performs the aforementioned feature engineering on all the second paths, obtains all new variables, and converts path data of each person into new variable data. In particular, in an embodiment, the processor 20 may pre-establish a comparison table for recording the correspondence between paths and variables. The processor 20 may generate a plurality of variables corresponding to the foregoing plurality of patterns according to the foregoing second paths and the comparison table.

The step of converting path data into new variable data is as follows: for a new variable X, if at least one path in the path data of a patient may generate X through the aforementioned process, in the new variable data of the patient, an X threshold is equal to 1, otherwise equal to 0.

The following is a path to illustrate the process of generating a new variable by feature engineering and the process of converting into path data.

### [Example of Step S301]

It is assumed that a path having a length of 3 is: A->B->C. Since the path length is 3, in step S301, a plurality of new variables is obtained from the foregoing patterns (1) to (7) by taking part or all of the diseases from 3 diseases (i.e., taking 1 disease, 3 diseases at most).

For the pattern V(1, PP, X), new variables may be obtained: A_1, B_2 and C_3. A number behind a baseline represents a position of a disease in the path.

For the pattern V(1, IP, X), new variables may be obtained: A, B and C.

For the pattern V(2, PP, PO), new variables may be obtained: A->B_1 and B->C_2. "->" represents an order. The variable "A->B_1" is taken as an example, where it represents that the order of suffering diseases is A->B, and A is at a first position in a path. For another example, the variable "B->C_2" represents that the order of suffering diseases is B->C, and B is at a second position in a path.

For the pattern V(2, PP, IO), new variables may be obtained: A&B_1&2, A&C_1&3 and B&C_2&3. The variable "A&B_1&2" is taken as an example, where it represents that diseases include A and B (in any order) and the two diseases are at first and second positions in a path. In other words, this example represents A->B or B->A from the first position in a path. For another example, the variable "A&C_1&3" represents that diseases include A and C (in any order) and the two diseases are at first and third positions in a path. In other words, the two positions (i.e., the first and third positions) may be A->C or C->A in a path. Other variables may be deduced by analogy, and the descriptions thereof are omitted herein.

For the pattern V(2, IP, PO), new variables may be obtained: A->B and B->C. The variable "A->B" is taken as an example, where it represents that diseases are sequentially A and B, but the positions of A and B in a path are not limited. For another example, the variable "B->C" represents that diseases are sequentially B and C, but the positions of B and C in a path are not limited. Other variables may be deduced by analogy, and the descriptions thereof are omitted herein.

For the pattern V(2, IP, IO), new variables may be obtained: A&B, A&C and B&C. The variable "A&B" is taken as an example, where it represents that diseases include A and B (in any order), and the positions of A and B in a path are not limited. For another example, the variable "A&C" represents that diseases include A and C (in any order), and the positions of A and C in a path are not limited. Other variables may be deduced by analogy, and the descriptions thereof are omitted herein.

For the pattern V(3, X, IO), a new variable may be obtained: A&B&C. The variable "A&B&C" is taken as an example, where it represents that diseases include A, B and C (in any order), and the positions of A, B and C in a path are not limited.

A total of 17 new variables may be obtained by the aforementioned patterns (1) to (7).

In an example, if there is a path A->B->C (threshold=1) in path data of patient B, the values of the aforementioned 17 new variables of patient B may be set to "1". In another example, if A->B->C field=0 and A->B->G field=1 in path data of patient C, since the path A->B->G may also generate new variables such as A, B, A->B and A&B, the values of the new variables related to A and B may also be set to "1".

Referring again to Fig. 3, after performing step S301, the processor 20 performs a step of filtering variables using a plurality of models (step S303). In more detail, the processor 20 may filter the plurality of variables generated in the foregoing step S301 using a plurality of models to obtain a plurality of optimal variables from the variables.

Fig. 4 is a flowchart of a multi-model variable filtering method in accordance with an embodiment of the present invention.

Referring to Fig. 4, the foregoing step S303 may be further subdivided into steps S401 to S405.

Referring to Fig. 4, in the step of filtering variables using a plurality of models, the processor 20 first determines a plurality of machine learning algorithms and a plurality of variable input patterns, and performs permutation and combination according to the determined machine learning algorithms and the variable input patterns to generate a plurality of models. Thereafter, the processor 20 may input the plurality of variables generated in the foregoing step S301 to the model generated in step S401 to obtain a post-filtering variable (step S401).

The variable input pattern refers to how variables are input to a machine learning model. A machine learning model (also referred to as One-model) may be established by inputting all variables once, and the variables are output using the model; or a plurality of models is established according to a variable pattern (also referred to as By-pattern) of an original variable, and a union operation is performed on output results of the plurality of models finally.

The following uses a random forest algorithm and a Logistic regression algorithm as an example. The processor 20 may generate a combination of the machine learning methods and the variable input patterns in Table 2 below:

**Table 2**

| Serial number of models | Machine learning method | Variable input pattern | Count of models |
|---|---|---|---|
| M01 | Random Forest | One-model | 1 |
| M02 | Random Forest | By-pattern | 7 |
| M03 | Logistic Regression | One-model | 1 |
| M04 | Logistic Regression | By-pattern | 7 |

In detail, in the step of generating a model in step S401, the processor 20 may establish a model (also referred to as a first model) for the aforementioned patterns (1) to (7), respectively, using a machine learning algorithm. Referring to Table 2, in the process of generating the first model, the processor 20 may establish models for the aforementioned patterns (1) to (7), respectively, using a random forest algorithm to generate seven models (i.e., model M02 in Table 2). For another example, the processor 20 may also establish models for the aforementioned patterns (1) to (7), respectively, using a Logistic regression algorithm to generate seven models (i.e., model M04 in Table 2).

In addition, in the step of generating a model in step S401, the processor 20 may generate a model (also referred to as a second model) corresponding to the aforementioned patterns (1) to (7) using a machine learning algorithm. Referring to Table 2, in the process of generating the second model, the processor 20 may establish a model (i.e., model M01 in Table 2) for the aforementioned patterns (1) to (7) using a random forest algorithm. In addition, the processor 20 may also establish models for the aforementioned patterns (1) to (7) using a Logistic regression algorithm to generate a model (i.e., model M03 in Table 2).

After obtaining the foregoing first model and second model, the processor 20 may input the plurality of variables obtained in step S301 to each of the foregoing first models (e.g., seven models in model M02 or seven models in model M04) to obtain post-filtering variables (also referred to as first post-filtering variables) output by each model. In other words, the seven models of model M02 is taken as an example, where there are seven groups of post-filtering variables. Thereafter, the processor 20 performs an union operation on the seven groups of post-filtering variables to obtain a group of post-filtering variables (also referred to as second post-filtering variables). Similarly, the seven models of model M04 is taken as an example, where there are seven groups of post-filtering variables. Thereafter, the processor 20 performs an union operation on the seven groups of post-filtering variables to obtain a group of post-filtering variables.

In addition, the processor 20 also inputs the plurality of variables obtained in step S301 to the foregoing second models to obtain post-filtering variables (also referred to as third post-filtering variables). For example, the model of model M01 is taken as an example, since model M01 includes only one model, there is only one group of post-filtering variables. Similarly, the model of model M03 is taken as an example, since model M03 includes only one model, there is only one group of post-filtering variables.

Thereafter, the processor 20 performs a performance prediction on the foregoing post-filtering variables (e.g., the second post-filtering variable and the third post-filtering variable) respectively using a plurality of third models (step S403) to select a variable having a better prediction accuracy rate therefrom as an optimal variable (step S405).

The foregoing models M01 to M04 are taken as an example, where four groups of post-filtering variables may be obtained after the foregoing step S401. In the step of performing the performance prediction on each group of post-filtering variables, the processor 20 establishes a plurality of models for each group of post-filtering variables using a plurality of machine learning methods, each model obtaining a prediction performance (e.g., prediction accuracy rate). These prediction performances are calculated statistically (e.g., averaging, maximizing, etc.) to obtain a performance representative of the group of post-filtering variables. Thereafter, in the process of selecting an optimal variable, the processor 20 compares the prediction performance of each group of post-filtering variables, and selects the best one (e.g., having the highest prediction performance) as an optimal variable.

Referring to Fig. 3 again, after performing step S303, the processor 20 restores the foregoing plurality of optimal variables to a path (also referred to as a third path) corresponding to the foregoing plurality of optimal variables (step S305). For example, the processor 20 restores the optimal variables to third paths corresponding to the optimal variables according to the comparison table used in the foregoing step S301.

The processor 20 may establish, after obtaining the third paths, a prediction model according to the third paths. Thereafter, when performing the risk assessment of dementia, the processor 20 may input a path to be predicted to the prediction model and output a probability of suffering a specific disease in the foregoing path to be predicted. The foregoing path to be predicted is composed of a plurality of diseases, and the count of the diseases is, for example, equal to the path length determined in the foregoing step S201.

An example of risk assessment for dementia may be as follows:
After obtaining the path to be predicted, the processor 20 uses, for example, historical data to calculate risk information such as a proportion of suffering dementia in the path to be predicted, and a proportion of the path to be predicted in a group of demented patients to all demented patients, and establishes a path and dementia risk information comparison table according to the foregoing information. Therefore, history data of any person is given and converted into path data, and then the risk of disease may be assessed using the comparison table. Table 3 below is a schematic diagram of a path and risk comparison table:

**Table 3**

| Path | Proportion of dementia in path | Total proportion of patients with dementia in path |
|---|---|---|
| Dizziness → anxiety → dementia | 52% | 4.3% |
| Coronary heart disease → stroke → dementia | 58% | 2.8% |

Another example of risk assessment for dementia may be as follows:
The processor 20 may establish prediction models for each group of post-filtering variables after the foregoing step S401, and take a prediction model of an optimal variable as an optimal model for generating a risk indicator such as a dementia probability or a prediction label. Thereafter, when the history of any person is given, the processor 20 may further convert a path into an optimal variable after converting into path data, and then use the optimal model to calculate a dementia risk indicator (e.g., probability or label).

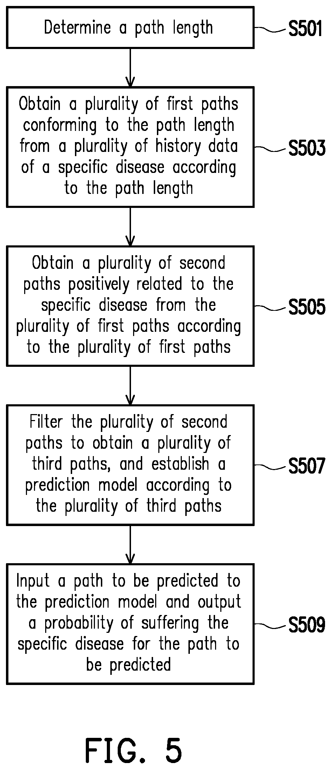

Fig. 5 is a flowchart of a disease suffering probability prediction method in accordance with an embodiment of the present invention.

Referring to Fig. 5, in step S501, a processor 20 determines a path length, where the path length is a count of diseases. In step S503, the processor 20 obtains a plurality of first paths conforming to the foregoing path length from a plurality of history data of a specific disease according to the foregoing path length, where the first path is composed of other diseases suffered sequentially before suffering the specific disease. In step S505, the processor 20 obtains a plurality of second paths positively related to the specific disease from the first paths according to the foregoing plurality of first paths. In step S507, the processor 20 filters the foregoing plurality of second paths to obtain a plurality of third paths, and establishes a prediction model according to the third paths. Finally, in step S509, the processor 20 inputs a path to be predicted to the prediction model and outputs a probability of suffering the specific disease for the path to be predicted, where the path to be predicted is composed of a plurality of diseases.

Based on the foregoing, the disease suffering probability prediction method and the electronic apparatus of the present invention can find an order (also referred to as a path) of diseases suffered before suffering a specific disease (e.g., dementia) from history data. People with these paths will have a higher probability of suffering the aforementioned specific disease in the future than those without them. In addition, the disease suffering probability prediction method of the present invention can further use the aforementioned path to calculate information such as a proportion or probability of suffering a specific disease.

## Claims

1. A disease suffering probability prediction method, applied to an electronic apparatus (100), the method comprising:
determining (S501) a path length, the path length being a count of diseases;
obtaining (S503) a plurality of first paths having the same length as the path length from a plurality of history data of a specific disease, each first path of the plurality of first paths being composed of other diseases suffered sequentially before suffering the specific disease;
obtaining (S505) a plurality of second paths positively related to the specific disease from the plurality of first paths by determining for each of the plurality of first paths using a machine learning method or a statistical method whether the first path is positively or negatively related to the specific disease;
filtering (S507) the plurality of second paths to obtain a plurality of third paths;
establishing a prediction model according to the plurality of third paths; and
inputting (S509) a path to be predicted to the prediction model and outputting a probability of suffering the specific disease for the path to be predicted, the path to be predicted being composed of a plurality of diseases,
wherein the step of filtering (S507) the plurality of second paths to obtain the plurality of third paths comprises:
generating (S301) a plurality of variables corresponding to a plurality of patterns according to the plurality of second paths by feature engineering;
filtering (S303) the plurality of variables using a plurality of models to obtain a plurality of optimal variables from the plurality of variables; and
restoring (S305) the plurality of optimal variables to the plurality of third paths corresponding to the plurality of optimal variables.

2. The disease suffering probability prediction method according to claim 1, wherein the plurality of patterns are related to permutation and combination of a position of a disease, an order of diseases and a count of diseases in each of the plurality of second paths.

3. The disease suffering probability prediction method according to claim 1, wherein the step of filtering (S303) the plurality of variables using the plurality of models to obtain the plurality of optimal variables from the plurality of variables comprises:
determining (S401) a machine learning algorithm;
determining (S401) a plurality of variable input patterns; and
generating (S401) the plurality of models according to the determined machine learning algorithm and the plurality of variable input patterns.

4. The disease suffering probability prediction method according to claim 3, wherein the step of generating (S401) the plurality of models according to the determined machine learning algorithm and the plurality of variable input patterns comprises:
establishing a plurality of first models for the plurality of patterns respectively using the machine learning algorithm; and
establishing a second model for the plurality of patterns using the machine learning algorithm.

5. The disease suffering probability prediction method according to claim 4, wherein the step of filtering (S303) the plurality of variables using the plurality of models to obtain the plurality of optimal variables from the plurality of variables comprises:
inputting the plurality of variables to the plurality of first models to obtain a first post-filtering variable output by each first model in the plurality of models, and performing a union operation on the first post-filtering variable output by each first model in the plurality of models to obtain a second post-filtering variable;
inputting the plurality of variables to the second model to obtain a third post-filtering variable; and
performing (S403) a performance prediction on the second post-filtering variable and the third post-filtering variable respectively using a plurality of third models to select (S405) a variable having a better prediction accuracy rate from the second post-filtering variable and the third post-filtering variable as the plurality of optimal variables.

6. The disease suffering probability prediction method according to claim 3, wherein the machine learning algorithm comprises a random forest algorithm and a Logistic regression algorithm.

7. The disease suffering probability prediction method according to claim 1, wherein the step of generating (S301) the plurality of variables corresponding to the plurality of patterns according to the plurality of second paths comprises:
generating the plurality of variables corresponding to the plurality of patterns according to the plurality of second paths and a comparison table.

8. The disease suffering probability prediction method according to claim 7, wherein the step of restoring (S305) the plurality of optimal variables to the plurality of third paths corresponding to the plurality of optimal variables comprises:
restoring the plurality of optimal variables to the plurality of third paths corresponding to the plurality of optimal variables according to the comparison table.

9. An electronic apparatus (100), comprising:
a storage circuit (24), recording a plurality of modules; and
a processor (20), accessing and executing the plurality of modules to perform the operations of the method according to claim 1.

## Patentansprüche

1. Verfahren zur Vorhersage der Erkrankungswahrscheinlichkeit, das auf eine elektronische Vorrichtung (100) angewendet wird, wobei das Verfahren umfasst:
Ermitteln (S501) einer Pfadlänge, wobei die Pfadlänge eine Anzahl von Krankheiten ist;
Ermitteln (S503) einer Vielzahl von ersten Pfaden, die dieselbe Länge wie die Pfadlänge aufweisen, aus einer Vielzahl von historischen Daten einer spezifischen Erkrankung, wobei jeder erste Pfad der Vielzahl von ersten Pfaden aus anderen Erkrankungen besteht, an denen der Patient nacheinander litt, bevor er an der spezifischen Erkrankung erkrankte;
Ermitteln (S505) einer Vielzahl von zweiten Pfaden, die in einem positiven Zusammenhang mit der spezifischen Erkrankung stehen, aus der Vielzahl der ersten Pfade, indem für jeden der Vielzahl der ersten Pfade unter Verwendung eines Verfahrens des maschinellen Lernens oder eines statistischen Verfahrens bestimmt wird, ob der erste Pfad in einem positiven oder negativen Zusammenhang mit der spezifischen Erkrankung steht;
Filtern (S507) der Vielzahl von zweiten Pfaden, um eine Vielzahl von dritten Pfaden zu erhalten;
Erstellen eines Vorhersagemodells auf der Grundlage der Vielzahl dritter Pfade; und
Eingeben (S509) eines vorherzusagenden Pfades in das Vorhersagemodell und Ausgeben einer Wahrscheinlichkeit, an der spezifischen Erkrankung zu erkranken, für den vorherzusagenden Pfad, wobei der vorherzusagende Pfad aus einer Vielzahl von Krankheiten besteht,
wobei der Schritt des Filterns (S507) der Vielzahl von zweiten Pfaden, um die Vielzahl von dritten Pfaden zu erhalten, umfasst:
Erzeugen (S301) einer Vielzahl von Variablen, die einer Vielzahl von Mustern entsprechend der Vielzahl von zweiten Pfaden entsprechen, durch Feature-Engineering;
Filtern (S303) der mehreren Variablen unter Verwendung mehrerer Modelle, um aus den mehreren Variablen mehrere optimale Variablen zu erhalten; und
Wiederherstellen (S305) der mehreren optimalen Variablen in die mehreren dritten Pfade, die den mehreren optimalen Variablen entsprechen.

2. Verfahren zur Vorhersage der Erkrankungswahrscheinlichkeit nach Anspruch 1, wobei die Vielzahl von Mustern sich auf Permutationen und Kombinationen einer Position einer Erkrankung, einer Reihenfolge von Erkrankungen und einer Anzahl von Erkrankungen in jedem der Vielzahl von zweiten Pfaden bezieht.

3. Verfahren zur Vorhersage der Erkrankungswahrscheinlichkeit nach Anspruch 1, wobei der Schritt des Filterns (S303) der Vielzahl von Variablen unter Verwendung der Vielzahl von Modellen, um die Vielzahl von optimalen Variablen aus der Vielzahl von Variablen zu erhalten, umfasst:
Bestimmen (S401) eines Algorithmus des maschinellen Lernens;
Festlegen (S401) einer Vielzahl von Variablen-Mustern; und
Erzeugen (S401) der mehreren Modelle gemäß dem festgelegten Algorithmus des maschinellen Lernens und den mehreren Musteren für Variablen.

4. Verfahren zur Vorhersage der Erkrankungswahrscheinlichkeit gemäß Anspruch 3, wobei der Schritt des Erzeugens (S401) der mehreren Modelle gemäß dem festgelegten Algorithmus des maschinellen Lernens und den mehreren Variableneingabemustern umfasst:
Erstellen einer Vielzahl von ersten Modellen für die Vielzahl von Mustern jeweils unter Verwendung des Algorithmus des maschinellen Lernens; und
Erstellen eines zweiten Modells für die Vielzahl von Mustern unter Verwendung des Algorithmus des maschinellen Lernens.

5. Verfahren zur Vorhersage der Erkrankungswahrscheinlichkeit nach Anspruch 4, wobei der Schritt des Filterns (S303) der mehreren Variablen unter Verwendung der mehreren Modelle, um die mehreren optimalen Variablen aus den mehreren Variablen zu erhalten, umfasst:
Eingeben der Vielzahl von Variablen in die Vielzahl von ersten Modellen, um eine erste nachgefilterte Variable zu erhalten, die von jedem ersten Modell in der Vielzahl von Modellen ausgegeben wird, und Durchführen einer Vereinigungsoperation an den ersten nachgefilterten Variablen, die von jedem ersten Modell in der Vielzahl von Modellen ausgegeben werden, um eine zweite nachgefilterte Variable zu erhalten;
Eingeben der mehreren Variablen in das zweite Modell, um eine dritte nachgefilterte Variable zu erhalten; und
Durchführen (S403) einer Leistungsprognose für die zweite nachgefilterte Variable bzw. die dritte nachgefilterte Variable unter Verwendung einer Vielzahl von dritten Modellen, um (S405) eine Variable mit einer besseren Prognosegenauigkeit aus der zweiten nachgefilterten Variable und der dritten nachgefilterten Variable als die Vielzahl von optimalen Variablen auszuwählen.

6. Verfahren zur Vorhersage der Erkrankungswahrscheinlichkeit nach Anspruch 3, wobei der Algorithmus des maschinellen Lernens einen Random-Forest-Algorithmus und einen logistischen Regressionsalgorithmus umfasst.

7. Verfahren zur Vorhersage der Erkrankungswahrscheinlichkeit nach Anspruch 1, wobei der Schritt des Erzeugens (S301) der mehreren Variablen, die den mehreren Mustern entsprechen, gemäß den mehreren zweiten Pfaden umfasst:
Erzeugen der mehreren Variablen, die den mehreren Mustern entsprechen, gemäß den mehreren zweiten Pfaden und einer Vergleichstabelle.

8. Verfahren zur Vorhersage der Erkrankungswahrscheinlichkeit nach Anspruch 7 , wobei der Schritt des Zuordnens (S305) der mehreren optimalen Variablen zu den mehreren dritten Pfaden, die den mehreren optimalen Variablen entsprechen, umfasst:
Wiederherstellen der Vielzahl optimaler Variablen auf die Vielzahl dritter Pfade, die der Vielzahl optimaler Variablen entsprechen, gemäß der Vergleichstabelle.

9. Elektronische Vorrichtung (100), umfassend:
eine Speicherschaltung (24), die eine Vielzahl von Modulen speichert; und
einen Prozessor (20), der auf die mehreren Module zugreift und diese ausführt, um die Schritte des Verfahrens gemäß Anspruch 1 durchzuführen.

## Revendications

1. Procédé de prédiction de la probabilité de souffrir d'une maladie, appliqué à un appareil électronique (100), le procédé comprenant :
la détermination (S501) d'une longueur de chemin, ladite longueur de chemin correspondant à un nombre de maladies ;
l'obtention (S503) d'une pluralité de premiers chemins ayant la même longueur que la longueur de chemin à partir d'une pluralité de données historiques relatives à une maladie spécifique, chaque premier chemin de la pluralité de premiers chemins étant composé d'autres maladies dont le sujet a souffert séquentiellement avant de souffrir de la maladie spécifique ;
l'obtention (S505) d'une pluralité de deuxièmes chemins présentant une relation positive avec la maladie spécifique à partir de la pluralité de premiers chemins, en déterminant, pour chacun des chemins de la pluralité de premiers chemins, à l'aide d'une méthode d'apprentissage automatique ou d'une méthode statistique, si le premier chemin est positivement ou négativement lié à la maladie spécifique ;
filtrer (S507) la pluralité de deuxièmes chemins afin d'obtenir une pluralité de troisièmes chemins ;
établir un modèle de prédiction en fonction de la pluralité de troisièmes chemins ; et
entrer (S509) un chemin à prédire dans le modèle de prédiction et fournir en sortie une probabilité de développer la maladie spécifique pour le chemin à prédire, le chemin à prédire étant composé d'une pluralité de maladies,
dans lequel l'étape consistant à filtrer (S507) la pluralité de deuxièmes chemins pour obtenir la pluralité de troisièmes chemins comprend :
générer (S301) une pluralité de variables correspondant à une pluralité de motifs en fonction de la pluralité de deuxièmes chemins par ingénierie des caractéristiques ;
le filtrage (S303) de la pluralité de variables à l'aide d'une pluralité de modèles afin d'obtenir une pluralité de variables optimales à partir de la pluralité de variables ; et
la réaffectation (S305) de la pluralité de variables optimales à la pluralité de troisièmes chemins correspondant à la pluralité de variables optimales.

2. Procédé de prédiction de la probabilité de souffrir d'une maladie selon la revendication 1, dans lequel la pluralité de motifs est liée à la permutation et à la combinaison d'une position d'une maladie, d'un ordre de maladies et d'un nombre de maladies dans chacun des chemins de la pluralité de deuxièmes chemins.

3. Procédé de prédiction de la probabilité de souffrir d'une maladie selon la revendication 1, dans lequel l'étape consistant à filtrer (S303) la pluralité de variables à l'aide de la pluralité de modèles afin d'obtenir la pluralité de variables optimales à partir de la pluralité de variables comprend :
la détermination (S401) d'un algorithme d'apprentissage automatique ;
la détermination (S401) d'une pluralité de motifs d'entrée variables ; et
générer (S401) la pluralité de modèles selon l'algorithme d'apprentissage automatique déterminé et la pluralité de modèles d'entrée de variables.

4. Procédé de prédiction de la probabilité de souffrir d'une maladie selon la revendication 3, dans lequel l'étape consistant à générer (S401) la pluralité de modèles selon l'algorithme d'apprentissage automatique déterminé et la pluralité de modèles d'entrée de variables comprend :
l'établissement d'une pluralité de premiers modèles pour la pluralité de motifs respectivement à l'aide de l'algorithme d'apprentissage automatique ; et
l'établissement d'un deuxième modèle pour la pluralité de motifs à l'aide de l'algorithme d'apprentissage automatique.

5. Procédé de prédiction de la probabilité de souffrir d'une maladie selon la revendication 4, dans lequel l'étape consistant à filtrer (S303) la pluralité de variables à l'aide de la pluralité de modèles afin d'obtenir la pluralité de variables optimales à partir de la pluralité de variables comprend :
l'entrée de la pluralité de variables dans la pluralité de premiers modèles afin d'obtenir une première variable post-filtrage produite par chaque premier modèle parmi la pluralité de modèles, et la réalisation d'une opération d'union sur la première variable post-filtrage produite par chaque premier modèle parmi la pluralité de modèles afin d'obtenir une deuxième variable post-filtrage ;
l'entrée de la pluralité de variables dans le deuxième modèle afin d'obtenir une troisième variable post-filtrage ; et
effectuer (S403) une prédiction de performance sur la deuxième variable post-filtrage et la troisième variable post-filtrage respectivement à l'aide d'une pluralité de troisièmes modèles afin de sélectionner (S405) une variable présentant un meilleur taux de précision de prédiction parmi la deuxième variable post-filtrage et la troisième variable post-filtrage en tant que pluralité de variables optimales.

6. Procédé de prédiction de la probabilité de souffrir d'une maladie selon la revendication 3, dans lequel l'algorithme d'apprentissage automatique comprend un algorithme de forêt aléatoire et un algorithme de régression logistique.

7. Procédé de prédiction de la probabilité de souffrir d'une maladie selon la revendication 1, dans lequel l'étape consistant à générer (S301) la pluralité de variables correspondant à la pluralité de motifs selon la pluralité de deuxièmes chemins comprend : :
la génération de la pluralité de variables correspondant à la pluralité de motifs selon la pluralité de deuxièmes chemins et un tableau de comparaison.

8. Procédé de prédiction de la probabilité de souffrir d'une maladie selon la revendication 7 , dans lequel l'étape consistant à réaffecter (S305) la pluralité de variables optimales à la pluralité de troisièmes chemins correspondant à la pluralité de variables optimales comprend :
la réaffectation de la pluralité de variables optimales aux pluralités de troisièmes chemins correspondant à la pluralité de variables optimales selon le tableau de comparaison.

9. Appareil électronique (100), comprenant :
un circuit de stockage (24), enregistrant une pluralité de modules ; et
un processeur (20), accédant à la pluralité de modules et les exécutant pour effectuer les opérations du procédé selon la revendication 1.
